# EUROPEAN PATENT APPLICATION

(11) **EP 2 308 970 A1**
(43) Date of publication of application: **13.04.2011**
(21) Application number: 09172463.3
(22) Date of filing: 07.10.2009
(51) Int. Cl.: C12N 15/00, C12N 15/52, C12P 1/06

(54) **Antibiotic compositions, methods for providing the same and Streptomyces coelicolor mutants for use in such methods**

(71) Applicant: Rijksuniversiteit Groningen, 9712 CP Groningen (NL)
(72) Inventor: Wietzorrek-Takano, Eriko, 9722 NR Groningen (NL); Gottelt, Marco, D-73230 Kirchheim (DE); Kol, Stefan, 9711 CL Groningen (NL)
(74) Representative: Hatzmann, Martin

(57) **Abstract**

The present invention relates to the field of preventive and therapeutic medicine. In particular, it relates to methods for providing microbial antibiotic extracts and compounds and use of those extracts and compounds in the treatment of a bacterial infection. Also provided are mutant strains of *Streptomyces coelicolor* and the use thereof in the production of an antibiotic composition.

## Description

The present invention relates to the field of preventive and therapeutic medicine. In particular, it relates to methods for providing microbial antibiotic extracts and compounds and uses thereof, among others in the treatment of a bacterial infection. It also relates to mutant *Streptomyces coelicolor* and uses thereof in providing antibiotic products.

The need for new antimicrobial agents is greater than ever because of the emergence of multidrug resistance in common pathogens, the rapid emergence of new infections, and the potential for use of multidrug-resistant agents in biological weapons. Paradoxically, major pharmaceutical companies are losing interest in the antibiotics market because these drugs may not be as profitable as drugs that treat chronic (long-term) conditions and lifestyle issues. For example, new antibacterial agents constitute only 6 of 506 drugs disclosed in the developmental programs of the largest pharmaceutical and biotechnology companies (Spellberg *et al.,* 2004 and Powers, 2004). In the four five-year periods between 1983 and 2002, the number of FDA approved novel antibiotic drugs has fallen by 56%, from 16 new antibiotics between 1983 and 1987 to just 7 new antibiotics between 1998 and 2002 (Spellberg *et al.,* 2004).

Infectious disease physicians are alarmed by the grim prospect that, in the near future, effective antibiotics may no longer be available to treat seriously ill patients. Especially the enterobacteriaceae, a family of Gram-negative bacteria comprising highly-infectious pathogens such as *Escherichia coli, Klebsiella pneumoniae, Staphylococcus aureus* and *Vibrio cholerae,* develop antibiotic resistance quicker than ever (Paterson, 2006; Boyle-Vavra and Daum, 2007; Slama, 2008).

Thus, there is a great demand for novel bioactive agents that are effective against pathogenic bacteria which are resistant to current antibiotics. The classical strategy for obtaining antibacterial agents is to screen microorganisms such as fungi (molds), actinomycetes (filamentous bacteria) and other bacteria for their capacity to produce such compounds.

Antibiotics obtained from actinomycetes are predominantly produced by streptomycetes, a family of mycelial soil bacteria with a complex life cycle and morphology. Streptomycetes are the richest source of antibiotics and other bioactive compounds used in medicine and agriculture (Hopwood, 2007). Estimates suggest up to several ten thousands of yet unidentified *Streptomyces* secondary metabolites of pharmaceutical interest (Watve *et al.,* 2001; Berdy, 2005). Streptomycetes are therefore of major interest in the search for new drugs.

In classical screening programs strains are isolated from their natural habitats and tested for production of antibiotic agents. Knowledge of the genetic background of secondary metabolite production allows the search for interesting genes. For example, genome analysis of the model streptomycete *Streptomyces coelicolor* A(3)2 revealed several secondary metabolite gene clusters (Bentley *et al.,* 2002), among them 11 well-characterised compounds including the three S. *coelicolor* antibiotics, the blue-pigmented polyketide (gamma-)actinorhodin (Act) (Rudd and Hopwood, 1979; Bystrykh *et al.,* 1996), the red-pigmented (undecyl-)prodigiosins (Red) (Feitelson *et al.,* 1985) and the calcium dependent antibiotic (CDA) (Hopwood and Wright, 1983; Lakey *et al.,* 1983). A fourth antibiotic, methylenomycin, is not encoded chromosomally as the others listed, but on a plasmid (SCP1) lacking in the model strain M145 (Bentley *et al.,* 2004). Furthermore, twelve additional "orphan" secondary metabolite clusters have been found to which no product has yet been assigned (Challis and Hopwood, 2003). The end products of other putative antibiotic gene clusters revealed in *Streptomyces* genome sequences, which are available in increasing numbers, also remain unknown (Challis, 2008). This is an undesired situation, as these gene clusters may encode novel and effective antibiotics.

It is likely that the end products could not yet be determined because their encoding gene clusters are "silent" or "dormant", i.e. the products are not expressed under standard laboratory conditions. For example, under standard conditions the genes encoding the antibiotics Act and Red are poorly expressed in most *Streptomyces* species. Species *S. lividans* does not produce Act even though it carries the entire biosynthetic gene clusters required for its production (Hopwood *et al.,* 1986; Lai *et al.,* 2002). This dormancy is not restricted to *Streptomyces* but can be observed in other bacteria as well. For example, *Bacillus subtilis* has the silent capability to produce the antibiotic neotrehalosediamine (3,3'-diamino-3,3'-dideoxy-α,β-trehalose or NTD) (Inaoka *et al.,* 2004).

Methods to activate antibiotic production in bacteria are known in the art. For example, it has been demonstrated that actinorhodin production in *S. coelicolor* and *S. lividans* can be activated by mutating the *rpoB* gene that encodes the B-subunit of RNA polymerase (Lai *et al.,* 2002). This mutation also activates NTD production in *B. subtilis* (Inaoka *et al.,* 2004). Actinorhodin production in *S. lividans* can also be activated by inserting a DNA fragment of *S. fradiae* encoding for a 132 nucleotides long transcript possibly acting as an antisense RNA (Romero *et al.,* 1992). Wang *et al.* (2008) describe a method for inducing multi-drug-resistance in *S. coelicolor* by introducing at least three drug resistance mutations. In Kim *et al.* (2001), actinorhodin production in *S. lividans* is activated by using glycerol as the sole carbon source in the culturing medium. Antibiotic production in *Streptomyces* can also be activated by deletion of the *scbA* gene that is involved in the biosynthesis of the γ-butyrolactone SCB1 (US 7,217,511; Butler et al., 2003) or by transposon mutagenesis (Baltz 1998; Baltz 2001).

The inventors surprisingly found that the production of two compounds, which have never been described in *S. coelicolor* before (the yellow pigment yCPK and the antibiotic abCPK), can be optimized by specific culturing conditions and/or by genetic alteration(s) of the *cpk* gene cluster of *S. coelicolor.* The *cpk* (*coelicolor* polyketide) cluster (formally named Kas cluster) contains a type I polyketide synthase and was described in detail in Pawlik *et al.,* 2007. Until the discovery of yCPK (M. Gottelt, submitted for publication), the cluster remained orphan. The yellow compound was found to be conditionally produced in *S. coelicolor* M145 and overproduced in mutant strain LW3 (M145 *scbR2::aac(3)*IV.) Strain LW3 carries a mutation in the *scbR2* gene that is located in the *cpk* cluster. The *scbR2* gene encodes for a γ-butyrolactone receptor homologue. The newly described compound yCPK was found to be related to the *cpk* cluster by comparison of production in a newly created mutant of the three classical chromosomally encoded secondary metabolites of *S. coelicolor* (Act, Red, CDA) and a quadruple mutant where also the *cpk* cluster was deleted.

The inventors surprisingly found that yCPK production in the model strain *S. coelicolor* was increased by the addition of glutamate to the medium. Under this condition, also an antibacterial agent (abCPK) was produced. Cell extracts comprising abCPK were found to be active against both Gram-positive and Gram-negative bacteria and the antibacterial agent was stable in methanol. The production of the antibiotic compound proved to be associated with genes of the *cpk* gene cluster by analysing mutants in the *act, red, cda* and the *cpk* gene clusters. Whereas in liquid culture yCPK was only found secreted into the medium, abCPK could be isolated from the cells which lacked yellow pigmentation. This implies that yCPK and abCPK are two distinct metabolites of the *cpk* biosynthetic pathway.

More surprisingly, the inventors found that production of the antibiotic compound by *S. coelicolor* can also be activated by the deletion of at least the *scF* gene in the *cpk* gene cluster. These mutants do not produce yCPK any longer, which is additional strong evidence that yCPK and abCPK are distinct from each other, and that antibacterial activity is independent from the yellow pigment and instead related to abCPK. Also with *S. coelicolor* mutants lacking a functional *scF* gene, production of abCPK could be further enhanced by supplementing glutamate to their culturing medium. The invention accordingly relates to methods for providing antibiotic extracts and compounds. Furthermore, it relates to use of those extracts and compounds in the treatment of a bacterial infection.

In one aspect, the invention relates to a method for providing an antibiotic composition e.g. an extract or (partially) purified antibiotic compound, comprising a) providing *Streptomyces coelicolor* or a mutant strain thereof capable of producing an antibiotic compound, b) culturing said strain under conditions allowing for production of an antibiotic compound, c) isolating at least one cultured cell, and d) preparing an extract from said isolated cell.

In one embodiment, step a) involves providing *Streptomyces coelicolor,* or a mutant strain thereof with a non-functional *scF* and/or *scbR2* gene, preferably in combination with functional *cpkA, cpkB, cpkC, cpkH, cpkJ, accA1, cpkF, cpkN, cpkK, scoT, scbA, scbB, scbC,* and *cpkO* genes. Preferably, step a) comprises providing *Streptomyces coelicolor* e.g. M145 or any other laboratory strain (herein also referred to as parental strain), or a mutant strain thereof wherein at least *scF* and/or *scbR2* has been disrupted or deleted and containing functional *cpkC, cpkH* and *cpkJ* genes. Although inactivation of the *scbR2* gene had a more pronounced effect on abCPK production, functional inactivation of the *scbR* gene is also envisaged. The mutant may overexpress one or more *cpk* genes, for example the *cpkF* and/or *cpkH* gene. When providing a mutant strain of *S. coelicolor,* at least one gene may be disrupted or deleted by using any gene disruption or deletion technique known in the art. For example, one or more genes in the *cpk* cluster are knocked out by homologous recombination with an artificially produced, non-functional allele. As used herein, the term "gene" is used broadly to refer to any segment of nucleic acids that is associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. The term "expressing" as used herein refer to the transcription and/or translation of an endogenous gene, open reading frame (ORF) or portion thereof in an organism such as *S. coelicolor.* Expression refers to the transcription and stable accumulation of sense (mRNA) or functional RNA. Expression may also refer to the production of at least one peptide or protein. The term "gene cluster" as used herein is defined as any set of two or more genes that serve to encode for the same or similar products, including sets of related genes that are arranged in more than one physical cluster. An "orphan" gene cluster refers to a gene cluster having one or more yet unknown functions or expressing one or more yet unknown products.

The term "mutant" refers to any organism or new genetic character arising or resulting from an instance of mutation in a parental strain. Accordingly, the term "mutation" as used herein is defined as any base-pair sequence change within the DNA of an organism resulting in the creation of a new character or trait not found in the parental strain. The terms "disrupting", "disruption", "deleting" and "deletion" as used herein refer to any method for at least partially disabling at least one functional gene in an organism. This includes deleting and knocking out at least one gene, whereby "knocking out" refers to any genetic technique in which an organism is engineered to carry genes that have been made inoperative (have been "knocked out" of the organism). This includes homologous recombination, for example with an artificially produced, non-functional allele such as a plasmid. The resulting organisms are referred to as "knockout organisms" or simply "knockouts". Also within the scope of the present invention is a mutant wherein one or more of the above genes is made non-functional by decreasing or abolishing gene expression (so-called "knock down" approach). For example, this can be achieved by gene silencing using antisense RNA that binds to mRNA.

The inventors found that *S. coelicolor* mutants containing non-functional *cpkC, cpkH* or *cpkJ* gene e.g. obtained by disrupting or deleting the *cpkC, cpkH* or *cpkJ* gene, were unable to provide antibiotic activity. Thus, for producing at least one antibiotic compound having the activity described above, a mutant requires functional *cphC, cpkH* and *cpkJ* genes. A mutant may have two or more than two mutations in the *cpk* gene cluster such as three, four or even five mutations. Thus, the mutant may be a polymutant such as a double, triple, quadruple or quintuple mutant.

Good results were observed when mutating at least the *scF* gene, which encodes a predicted extracellular oxidoreductase. Mutant strain LW38 (M145 *scF::aac(3)*IV), wherein the *scF* gene was deleted, failed to produce yCPK but (over-)produced abCPK, as determined by strains grown on solid medium plate and in isolated cell extracts. This suggests that the ScF protein is involved in the conversion of abCPK to yCPK.

The inventors found that *scbR2* mutant strain LW3 not only overproduces the yellow pigment, but also overproduces the antibiotic compared to the parental strain.

A mutant strain containing functional *cphC, cpkH* and *cpkJ* genes may also have a disruption or deletion in at least one gene located inside the *cpk* cluster and at least one gene located outside the *cpk* cluster. For example, as described in US 7,217,511 and Butler *et al.,* 2003 the inventors previously found that mutating the *scbA* gene of *S. coelicolor* results in increased production of Act and Red. The *scbA* gene encodes a homologue of A-factor synthetase (AfsA) of *S. griseus.* For example, the mutant is a double mutant and has a disruption or a deletion in the *scF* and *scbR2* genes. In a specific aspect, the mutant overexpresses the putative resistance gene *cphF* in combination with non-functional *scF* and *scbR2* genes. As another example, the mutant is a polymutant and has a deletion in the *scF* gene, the *scoT* gene, the *cpkE* gene and in at least one gene in the γ-butyrolactone gene cluster. Also of interest are genes in other known or cryptic secondary metabolite gene clusters (see Bentley, 2002). Similar to the *act red cda* mutants, blocking other putatively "competing" secondary metabolite biosynthesis pathways may lead to the overproduction of the (antibiotic) CPK products.

In another aspect the invention provides a mutant strain of *Streptomyces coelicolor,* characterized in that at least the *scF* gene is non-functional e.g. has been disrupted, deleted or knocked down. It can be of further advantage to delete or disrupt one or more other genes, such as *scbR2* and/or *scbR.* The mutant preferably has functional *cpkA, cpkB, cpkC, cpkH, cpkJ, accA1, cpkF, cpkN, cpkK, scoT, scbA, scbB, scbC* and *cpkO* genes. Overexpression of one or more of these functional genes, for example *cpkF* and/or *cpkH,* may be desirable with respect to antibiotic production.

In one embodiment, the mutant has non-functional *scF* and *scbR2* genes, optionally in combination with overexpression of the putative resistance gene *cpkF.* In another aspect the invention provides the use of a mutant strain of *Streptomyces coelicolor* in the preparation of an antibiotic extract or compound, wherein the mutant strain is characterized in that its *scF* and *scbR2* genes are non-functional and the mutant strain contains functional *cpkA, cpkB, cpkC, cpkH, cphJ, accA1, cpkF, cpkN cpkK scoT, scbA, scbB, scbC* and *cpkO* genes. For example, M145 *scF::aac(3)*IV is cultured under conditions allowing for the production of an antibiotic compound, after which cells are extracted from the culture and an antibiotic compound is isolated from the cell extract.

Step b) of a method as provided herein comprises the culturing of a (mutant) *S. coelicolor* strain or mixture of two or more strains under conditions suitable or optimized for the production of an antibiotic. In one embodiment, the culturing in step b) of the method is performed in the presence of glutamate or a salt thereof. The terms "glutamate" and "Glu" as used herein refer to any carboxylate anion or salt of glutamic acid (C₅H₉NO₄, also known as 2-aminopentanedioic acid), and any salt thereof. This includes glutamate salts known in the art as monosodium glutamate and L-glutamic acid monosodium salt. Methods for supplementing glutamate or a salt thereof are known to a person skilled in the art. The glutamate or salt thereof may be supplemented to a culture directly, for example by pouring a glutamate-comprising solution directly on the culture, or supplemented indirectly, such as by adding glutamate to the medium before inoculation. The glutamate or salt thereof is supplemented to the culture medium prior to or after inoculation. Preferably, it is added before inoculation.

A skilled person can readily determine suitable amounts of glutamate or a salt thereof to activate or enhance antibiotic production in *S. coelicolor.* The inventors found a linear correlation between the amount of supplemented glutamate and the final production level of the antibiotic compound abCPK. Thus, increased supplementation of glutamate or a salt thereof results in increased production of abCPK.

Production of abCPK by *S. coelicolor* could be observed in the presence of glutamate at a concentration of around 50 mM or higher. Therefore, in one embodiment glutamate or a salt thereof is present in a concentration of at least 50 mM, for example between 100 mM and 3 M. For example, a culture of *S. coelicolor* is supplemented with 500 mM glutamate. In another embodiment, glutamate or a salt thereof is present in a concentration of up to 2 M, preferably up to 1 M. For example, glutamate is present in a concentration of 1.5 M. Very good results were observed when glutamate was present in a concentration of more than 300 mM. Therefore, in a preferred embodiment glutamate or a salt thereof is present in a concentration of at least 300 mM. For example, a mutant strain of *S. coelicolor* having a non-functional *scF* gene is cultured in the presence of about 300 to 350 mM, like 325 mM L-glutamic acid monosodium salt.

It will be appreciated by a person skilled in the art that selecting a suitable medium is important when culturing a microorganism such as a bacterium. Many bacteria require special media for optimized growth and/or production, and not choosing the optimal medium may result in retarded growth and/or loss or even absence of production. The term "medium" refers to any solid, semisolid or liquid carrier such as an agar plate, an agar gel or a broth that is suitable for culturing a microorganism such as a bacterium. A medium generally comprises a carbon source such as a sugar, a nitrogen source such as an amino acid, water and at least one micronutrient such as a salt or a trace metal providing essential elements such as magnesium, nitrogen, phosphorus, sulfur, copper and iron. A medium may be defined (also known as chemically defined medium), i.e. the amount of each ingredient is known. A medium may also be undefined (also known as basal or complex medium), i.e. the amount of each ingredient is unknown. This includes any nutrient medium, minimal medium, supplementary minimal medium, selective medium and differential medium (also known as indicator medium) such as for example, but not limited to, Difco™ Nutrient Agar, Difco™ Nutrient Broth, Supplemental Minimal Media (SMM), SMM comprising 1.5% (w/v) agar (also known as SMMS), eosin-methylene blue agar (EMB), blood agar, Luria or Luria-Bertani broth (LB), LB agar plates, MacConkey agar, hektoen enteric agar (HE), Terrific Broth (TB), Trypton Soya Broth (TSB), soft nutrient afar (SNA), X-gal plates, chocolate agar, Mitis Salivarius agar (MS agar), Super Optimal Broth (SOB), A1 broth and plate count agar.

The culturing medium used in step b) of the method of the invention may be a solid, semisolid or liquid medium. When using a solid or semisolid medium such as an agar gel, best results are obtained when the glutamate or salt thereof is supplemented during the preparation of the medium, before it has set. When using a liquid medium such as a broth or a suspension, the glutamate or salt thereof may be added directly to the medium under mixing or stirring. A skilled person may readily determine suitable media for culturing *S. coelicolor* or a mutant strain thereof under conditions allowing for production of an antibiotic compound. When culturing on a solid medium, good results were obtained using Difco™ Nutrient Agar (also known as DNAgar) or Supplemental Minimal Media (SMM) comprising 1.5% (w/v) agar (also known as SMMS) with addition of glutamate. For example, parental or mutant *S. coelicolor* is cultured on DNAgar supplemented with 200 mM glutamate. When culturing in a liquid medium, good results were obtained using Difco™ Nutrient Broth (also known as DNBroth). For example, strain LW38 is cultured in DNBroth supplemented with 325 mM glutamate. Therefore, preferably a *S. coelicolor* strain is cultured under conditions allowing for production of an antibiotic compound, wherein the conditions comprise the use of Nutrient Agar, Nutrient Broth or Supplemental Minimal Media comprising 1.5% (w/v) agar as culturing medium. For example, a *scF* mutant is cultured in Supplemental Minimal Media comprising 1.5% (w/v) agar, optionally in the presence of glutamate.

The yellow pigment yCPK was only found to be secreted into the medium. In contrast, antibiotic extracts comprising abCPK could be isolated best from the cultured cells (lacking yellow pigmentation by yCPK). Therefore, a method according to the invention comprises isolating at least one cultured cell, as recited in step c). Cultured cells can be isolated using techniques that are known in the art. For example, cells cultured in a liquid medium can be separated from the medium by filtration over a membrane. Good results were obtained when cells were cultured in a liquid medium such as Difco™ Nutrient Broth and isolated by centrifugation. After centrifugation, the supernatant may be discarded.

Step d) of a method according to the invention comprises preparing an extract from the isolated cells. The extract may be prepared by any method deemed suitable by a person skilled in the art. The preparation may involve disrupting the cell walls, for example by subjecting the cells to a lysing enzyme such as lysozym, lysostaphin, labiase and the like or physical disruption using French press or sonication . For example, a culture of a *scF* mutant is isolated from a medium by using centrifugation and then opened with French press. The preparation may also involve one or more purifying steps such as washing, filtration, distillation, fractionation, adsorption and the like, for instance for removing cell material such as debri, sugars and lipids. For example, a cell isolate is extracted for the compound and then run over a chromatography column. When using centrifugation to isolate the cells, the obtained cell pellet may be resuspended, preferably in at least one extraction solvent. The inventors found that polar extraction solvents such as an alcohol (for example ethanol or methanol), dimethyl sulfoxide (DMSO), dimethyl formamide (DMF), dimethyl acetamide (DMAc), 1,3-dimethyl-3,4,5,6-tetrahydro-2(1H)-pyrimidinone (DMPU) and the like are particularly suitable for extracting abCPK from cultured cells. Therefore, in one embodiment the methods according to the invention comprise the use of alcohol, dimethyl sulfoxide, dimethyl formamide, dimethyl acetamide and/or 1,3-dimethyl-3,4,5,6-tetrahydro-2(1H)-pyrimidinone when extracting an antibiotic from cultured cells. For example, cells of *S. coelicolor* that were grown under conditions allowing for antibiotic production are isolated by means of centrifugation and an antibiotic extract is prepared by pellet resuspension using methanol.

The inventors surprisingly found that the extract is bioactive against both Gram-positive and Gram-negative bacteria. Thus, in another aspect the invention provides an antibiotic extract that is obtainable by a method according to the invention, characterized in that it is biologically active against Gram-positive and Gram-negative bacteria. The extract may be used in a dry form such as a powder or in (semi)liquid form such as a concentrate, a solution or a suspension. The extract may be used for any purpose deemed suitable by a skilled person, such as for isolating a bioactive compound or for the manufacture of a medicament. For example, a medicine for treating a bacterial infection is prepared from an antibiotic extract of *S. coelicolor* as disclosed herein.

In yet another aspect, the invention provides a method for providing an antibiotic compound, comprising i) providing an antibiotic extract obtainable by a method for providing an antibiotic extract as described hereinabove, and ii) isolating at least one antibiotic compound from said extract. An antibiotic compound can for instance be isolated from the extract by applying one or more isolation techniques such as distillation, fractionation, crystallization, stripping, extraction, filtration, adsorption, ion exchange, chromatography and the like. For example, High Performance Liquid Chromatography (HPLC) or Liquid Chromatography - Mass Spectrometry (LC-MS) is used to isolate an antibiotic compound from a (methanolic) extract prepared from *scF* deficient cells.

The isolation step may optionally be followed by the use of one or more analytical methods such as X-Ray crystallography, NMR, spectroscopy or Matrix-assisted laser desorption/ionization - Time of Flight (MALDI-TOF) to analyze the structure of the at least one antibiotic compound.

The invention also provides an antibiotic compound obtainable by that method, characterized in that it is biologically active against Gram-positive and Gram-negative bacteria. Microorganisms such as bacteria produce many different types of primary and secondary metabolites such as peptides, non-peptidic compounds, ribosomal compounds and nonribosomal compounds, for example nonribosomal peptides. The terms "peptide" and "protein" are used interchangeably and are defined as any polymer formed from amino acids that are linked in a defined order by amide bonds or peptide bonds. This includes any ribosomal peptide, non-ribosomal peptide, peptone, monopeptide, dipeptide, tripeptide, oligopeptide, polypeptide and peptide fragment. The term "metabolite" as used herein refers to any intermediate or product of a metabolic pathway. This includes any primary metabolite, i.e. a metabolite that is directly involved in normal growth, development or and reproduction, and any secondary metabolite, i.e. a metabolite that is not directly involved in those processes but usually has an important ecological function such as an antibiotic.

The chemical structure of the compound(s) may comprise an aliphatic, unsaturated C₁₂ polyketide backbone that is formed from six C2 units derived from acetate (Pawlik *et al*., 2007). The backbone may be modified by tailoring enzymes of the *cpk* cluster.

By running an extract of cultured cells over size fractionation columns, the inventors found that both yCPK and the antibiotic compound abCPK have a molecular size that is smaller than 10 kDa. Accordingly, the antibiotic compound is smaller than 10 kDa.

The antibiotic extract or compound proved not only to be active against Gram-positive bacteria such as *Bacillus subtilis* and *Micrococcus luteus,* but also against Gram-negative bacteria like *Escherichia coli.* Thus, provided is an antibiotic composition (e.g. extract or compound) that is effective against both Gram-positive and Gram-negative bacteria. It can thus be used for treating infections caused by such bacteria. Therefore, in another aspect the invention provides an extract or compound obtained from S. *coelicolor* for use as a medicament. In one embodiment, the extract or compound is provided for the treatment of a bacterial infection. The bacterial infection may be caused by a Gram-positive bacterium, for example *B. subtilis* or *M. luteus,* and/or a Gram-negative bacterium, for example *E. coli.*

The extract or compound can be used in the preparation of a medicament for the treatment of a bacterial infection. The medicament may be administered to a subject in need thereof by any means known to be suitable to those skilled in the art.

Also provided is a package, container or spray container comprising an extract or compound according to the invention. Exemplary packages include sachets, plastic containers, (squeeze) tubes and a sprayer. The package, container or spray container may contain instructions for use.

According to another aspect of the invention, a method for treating or preventing a bacterial infection in a subject in need thereof is provided, comprising administering to said subject an effective dosage amount of an antibiotic extract or compound. The effective dosage amount may be administered via any route. For example, an antibiotic extract obtained from *S. coelicolor* is administered topically to a subject suffering from an infection caused by *E. coli.*

Effective amounts of the antibiotic extract or compound are amounts that are sufficient to bring about an efficacious effect against symptoms associated with a bacterial infection, and can be determined by those skilled in the art by routine experimentation. Furthermore, professional guidelines on the use of antibiotics in various indications exist and their use has been described in handbooks (e.g. AHFS™ Drug Information® or Martindale, The Complete Drug Reference).

Antibiotics are widely used as a selective agent, e.g. in a research or diagnostic setting. For example, the classical plasmid transformation protocol for plants involves a selective test wherein transformed plant embryos are subjected to a bioactive agent such as kanamycin, geneticin, hygromycin and paromomycin sulfate. Antibiotics such as vancomycin and tetracycline are commonly used as selective agent for isolating drug resistant bacteria species.

Therefore, the invention also provides an extract or a compound for use as a selective agent. In one embodiment, the extract or compound is used as a selective agent for isolating a bacterial strain. For example, abCPK is used to isolate resistant strains of *E. coli, B. subtilis, M. luteus* by use of a resistant gene to cloned fragments of DNA in these strains. In another embodiment, the extract or compound is used as a selective agent for selecting transformed plant embryos. For example, the extract or compound is used for selecting genetically transformed embryos of a plant or crop such as wheat, corn or potato. Other uses include the selection for abCPK resistant putative antibiotic producing organisms such as other streptomycetes. Resistance than could indicate a similar resistance mechanism as the one for abCPK in S. *coelicolor.* This may indicate the potential of the selected organism to produce an antibiotic similar to abCPK since the unknown antibiotic in the selected strain is prone to a resistance mechanism similar to that for abCPK. Like that, novel antibiotics similar to abCPK and/or with a putatively similar structure can be detected.

The invention is exemplified by the following examples.

### LEGENDS TO THE FIGURES

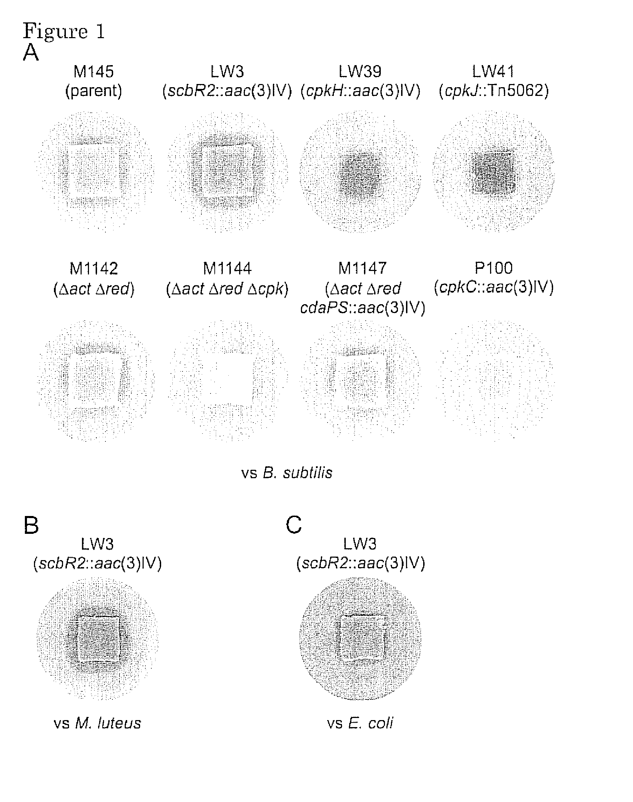

Figure 1: *cpk* cluster related bioactivity of *S. coelicolor* against *B. subtilis, M. luteus* and *E. coli* and. Panel A shows bioactivity of *S. coelicolor* strains M145 (parent), LW3 (M145 *scbR2::aac(3)IV),* M1142 (M145 *Δact Δred*), M1144 (M145 *Δact Δred Δcpk*), M1147 (M145 *Δact Δred cdaPS::aac(3)*IV) and absent bioactivity of P100 (M145 *cpkC::aac(3)*IV), LW39 (M145 *cpkH::aac(3)*IV) and LW41 (M145 *cpkJ.:Tn5062*) against *B. subtilis.* Panel B and C show bioactivity of strain LW3 against *M. luteus* against *E. coli,* respectively.

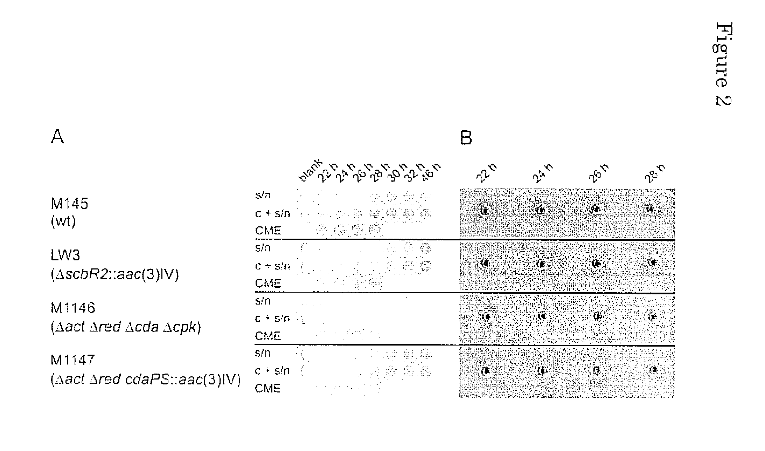

Figure 2: *cpk* gene cluster dependent production of an antibacterial compound in glutamate-supplemented liquid culture by *S. coelicolor* strains M145 (parent), LW3 (M145 *scbR2::aac(3)*IV), M1146 (M145 *Δact Δred Δcpk Δcda*) and M1147 (M145 *Δact Δred cdaPS::aac(3)*IV). Panel A shows cell-free culture supernatant (s/n) and cells in culture medium (c + s/n), and cell methanol extracts (CME). Culture medium is given as blank reference sample. Panel B shows biological activity, depicted as growth inhibition of *B. subtilis.* Pictures were taken from the top.

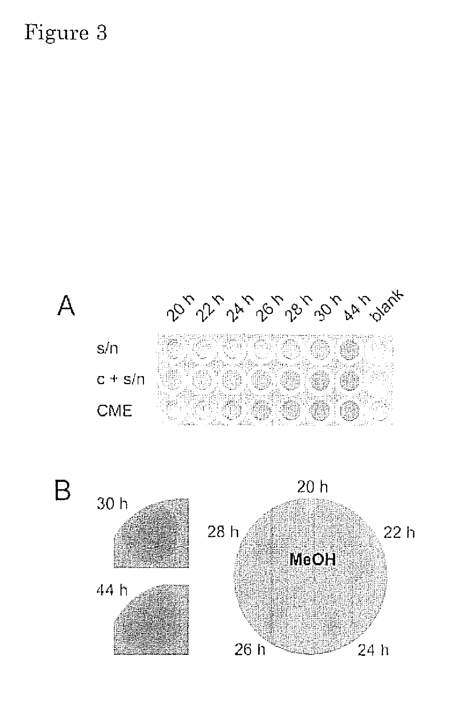

Figure 3: Optimised extraction and bioactivity test of the antibiotic CPK compound (abCPK). Panel A shows cell-free culture supernatant (s/n), cells in culture medium (c + s/n) and cell methanol extracts (CME) of *S. coelicolor* strain LW3 (M145 *scbR2::aac(3)IV).* Panel B shows antibiotic activity of CME obtained from strain LW3 against *B. subtilis* at different timepoints of extraction.

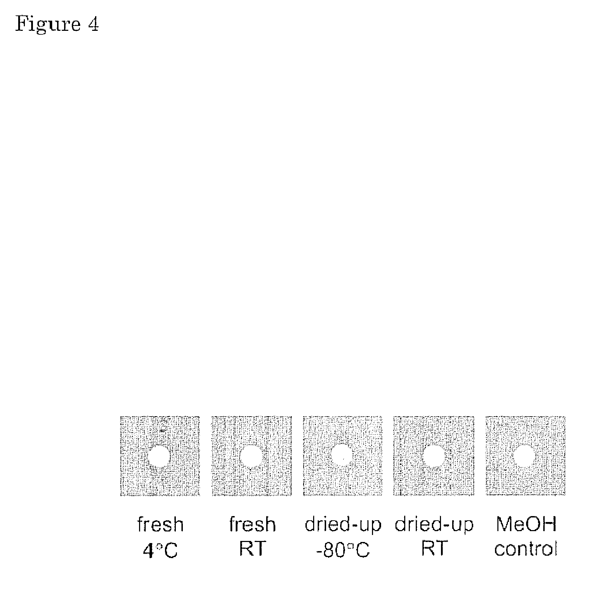

Figure 4: Stability of the antibiotic CPK compound in cell methanol extracts (CMEs) from *S. coelicolor* strain LW3 (M145 *scbR2::aac(3)IV),* depicted by bioactivity against *B. subtilis* after storage of the CMEs at 4°C, room temperature (RT) or -80°C. Pictures were taken from the bottom of the plate.

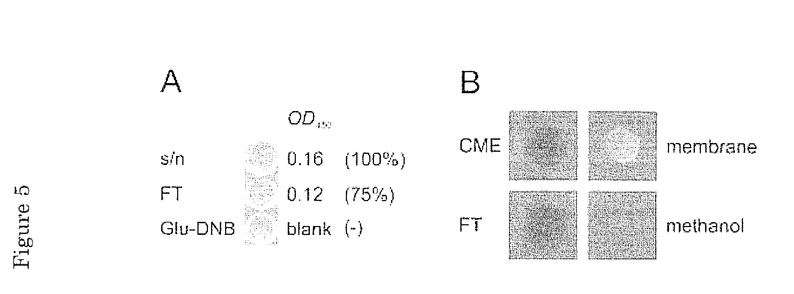

Figure 5: Molecular mass determination of *cpk* gene cluster products. Supernatant from a 28 h *S. coelicolor* M145 Glu-DNB culture (A) and *S. coelicolor* LW3 cell methanol extract (CME) from a 28 h Glu-DNB culture sample (B) were spun through a 10 kDa size fractionation column. The yellow-pigmented yCPK was detected in the culture supernatant (s/n) and the column flow-through (FT) by OD₄₅₀ determination against a blank culture medium reference (Glu-DNB). Results are given on the right, numbers in brackets indicate relative OD₄₅₀ values (A). The antibiotic abCPK was detected by bioactivity assays against *B. subtilis* in fresh CME and the column flow-through (FT). Also the size fractionation membrane was applied to the activity test, as well as a methanol control. To determine inhibition of bacterial growth, the indicator plate was incubated at 30°C overnight and pictures were taken from the bottom (B).

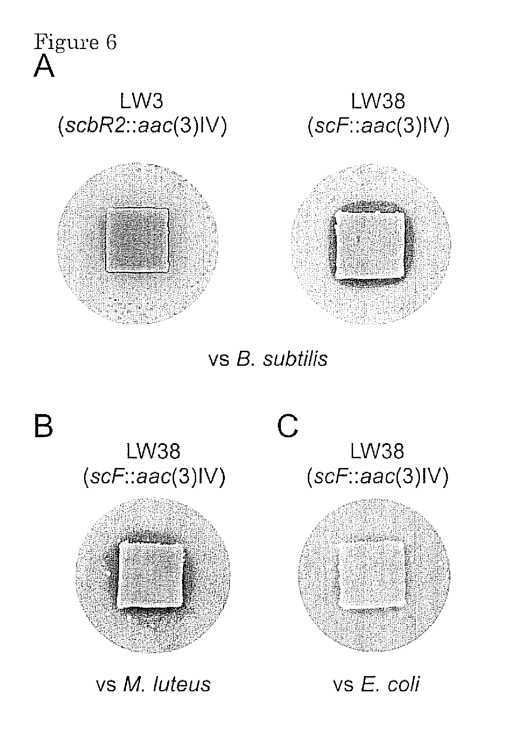

Figure 6: Bioactivity of *S. coelicolor* LW38. Panel A shows increased bioactivity of *S. coelicolor* strain LW38 (M145 *scF::aac(3)*IV) against *B. subtilis* compared to strain LW3 (M145 *scbR2::aac(3)*IV). Panel B and C show bioactivity of strain LW38 against *M. luteus* and *E. coli,* respectively.

### EXAMPLE 1: S. coelicolor produces an antibiotic compound related to the cpk gene cluster and its production is enhanced by glutamate

*S. coelicolor* has previously been shown to produce secondary metabolites with antibiotic activity (e.g. the three classical *S. coelicolor* antibiotics Act, Red and CDA). It was tested whether a *cpk* gene cluster product has any antibacterial activity. Therefore, several mutant *S. coelicolor* strains were grown for 27 hours on Glu-DNAgar plates. The plates were then completely overlaid with Soft Nutrient Agar containing 0.4% of the indicator strains *Bacillus subtilis, Micrococcus luteus* and *Escherichia coli* and further incubated overnight as described below.

### Bacterial strains and growth conditions

Bacterial strains and plasmids used are listed in Table 1. *Streptomyces* was manipulated as previously described (Kieser *et al., 2000*). *E. coli* strain JM101, strain ATCC 9341 (*Micrococcus luteus,* reclassified to *Kocuria rhizophila* (Tang and Gillevet, 2003), *Bacillus subtilis* DB104 (Kawamura and Doi, 1984) and strain ATCC 6633 (*Bacillus subtilis* subsp. *spizizenii*) were cultured at 30 °C in LB medium (Sambrook *et al.,* 2001). Difco™ Nutrient Agar (DNAgar) and Difco™ Nutrient Broth (DNBroth or DNB) are described in Kieser *et al.,* 2000. SMMS is the agar (1.5%, w/v) version of SMM (Kieser *et al.,* 2000) as described by Takano *et al.* (2001). In some cases 10 to 400 mM (final conc.) of L-glutamic acid monosodium salt (glutamate or Glu) from Sigma was added to DNB, DNAgar and SMMS additionally or to replace the casaminoacids contained in standard SMMS. Typically, 325 mM Glu was used. MS agar (Kieser *et al.,* 2000) was used to grow cultures for preparing spore suspensions, the spore concentration was determined on LB plates. Soft nutrient agar (SNA) (Kieser *et al.,* 2000) was used for antibacterial activity tests.

**Table 1. Bacterial strains and plasmids used.**

| Bacterial strains | Comments | References | SCO number of the deleted / disrupted genes* |
|---|---|---|---|
| *Bacillus subtilis* | | | |
| ATCC 6633 | - | Nakamura *et al.,* 1999 | *-* |
| DB104 | - | Kawamura and Doi, 1984 | - |

| *Escherichia coli* | | | |
|---|---|---|---|
| JM101 | - | Sambrook and Russel, 2001 | - |

| *Micrococcus luteus* | | | |
|---|---|---|---|
| ATCC 9341 | (reclassified to *Kocuria rhizophila*) | Tang and Gillevet, 2003 | - |

| *Streptomyces coelicolor* A3(2) | | | |
|---|---|---|---|
| M145 | A3(2) SCP1-SCP2-derivative | Kieser *et al.,* 2000 | - |
| M1142 | M145 *Δact Δred* | *-* | *-* |
| M1144 | M145 *Δact Δred Δcpk* | *-* | - |
| M1146 | M145 *ΔactdΔ red Δcda Δcpk* | - | - |
| M1147 | M145 *ΔactdΔ red cdaPS::aac(3)*IV | - | - |
| M1148 | M145 *Δact Δred Δcda* | - | - |
| M1157 | M1148 *rpoB* | - | SCO4654 |
| LW3 | M145 *scbR2::aac(3)*IV | - | SCO6286 |
| LW38 | M145 *scF::aac(3)*IV | - | SCO6272 |
| LW39 | M145 *cpkH::aac(3)*IV | - | SCO6281 |
| LW41 | M145 *cpkJ*::Tn5062 | - | SCO6283 |
| LW42 | M1157 *Δact Δred* | - | - |
| LW43 | M1157 *cpkH::aac(3)*IV | - | SCO6281 |
| LW44 | M1157 *scbR2::aac(3)*IV | - | SCO6286 |
| P100 | M145 *cpkC::aac(3)*IV | Pawlik *et al.,* 2007 | SCO6273 |

| | | | |
|---|---|---|---|
| * numbers according to Bentley *et al*., 2002; for a SCO number list of all *cpk* gene cluster genes see Table 1 in Pawlik *et al*., 2006 | | | |

### Secondary metabolite production and antibacterial activity assay in solid culture

For solid cultures, each 4 x 10⁷ spores from -20 °C spore stocks in 20% glycerol of *S. coelicolor* were streaked out for a 2.5 x 2.5 cm square or an irregular patch on DNAgar and SMMS plates (supplemented with glutamate) and incubated at 30 °C in darkness. For the antibacterial activity assay, plates were overlaid after 27 hours of growth with 2 ml SNA containing 0.4% of the indicator strains *E. coli, B. subtilis* or *M. luteus* from overnight cultures in 3 ml LB medium where the SNA completely covers the patch of *Streptomyces.* After further incubation overnight at 30 °C pictures were taken from the bottom to determine zones of bacterial growth inhibition.

### Results and conclusions

Growth of *B. subtilis* was inhibited with strains M145, LW3, M1142, and M1147, but not with the other *cpk* mutant strains LW41, M1144 and P100 (Fig. 1A). Antibacterial activity seemed to be increased in the *scbR2* mutant strain LW3 when compared to the parental strain M145 (Fig. 1A). Identical results were obtained using the Gram-positive bacterium *M. luteus,* and the Gram-negative bacterium *E. coli* as indicator strains (results for LW3 are shown in Fig. 1B and 1C). Therefore, it is concluded that production of the antibacterial CPK compound (abCPK) is related to the *cpk* gene cluster.

In a further step, a knockout mutant of *scF* in the *cpk* cluster was prepared using the Redirect system (Gust *et al.,* 2003). The *scF* gene encodes a predicted extracellular oxidoreductase and might be involved in one of the final steps of the biosynthesis of the CPK end product. Red and Act production in the resulting strain (LW38 (M145 *scF::aac(3)*IV)) was first detected after 47 and 96 hours, respectively (data not shown), and resembles that of the *cpkC* mutant strain P100 (Fig. 1A). In contrast to P100, LW38 still inhibited growth of the indicator strains *E. coli, M. luteus* and *B. subtilis* (Fig. 1), suggesting that the ScF protein may have a role in the conversion of abCPK into yCPK. In agreement with this, LW38 may show a higher antibiotic activity than the parental strain M145 (Fig. 1A). These observations correspond with a model where a bioactive compound is first transported outside of the cell and is then modified to form the yCPK compound.

Mutants LW39 and LW41 were also created using the Redirect system or by transposon mutagenesis, respectively (see Table 1). Strain LW39 contains a disruption of the *cpkH* gene, which is homologous to the aforementioned *scF* gene. The two homologues show 53 and 49% identity to AclO, a putative oxidoreductase involved in aclacinomycin production in *Streptomyces galilaeus.* They are described as secreted FAD-binding proteins (Pawlik *et al.,* 2006) and have been shown to be substrates of the *S*. *coelicolor* Tat protein export system (Widdick *et al.,* 2001). Strain LW41 contains a disruption of the *cpkJ* gene, which is homologous to nucleoside-diphosphate-sugar epimerases. In both LW39 and LW41 no antibiotic activity was detected, suggesting a disturbed production of abCPK (Fig. 1A). Thus, not every possible genetic disruption or deletion in the *cpk* gene cluster of *S. coelicolor* may be successful in providing antibiotic compounds.

Some bioactivity against *B. subtilis* and *M. luteus* could be observed in reference DNAgar cultures, i.e. DNAgar cultures without supplemental glutamate. However, the inhibition zones were only very small when compared to the inhibition zones that were observed on glutamate-supplemented DNAgar plates. More importantly, no activity could be observed against *E. coli* (data not shown).

Thus, addition of glutamate to the medium of *S. coelicolor* cultures is advantageously used to increase their CPK related bioactivity.

### EXAMPLE 2: An antibiotic CPK compound is produced by strains of S. coelicolor in liquid cultures and can be extracted from cells with methanol

To isolate the antibiotic CPK compound of *S. coelicolor* and mutants thereof, cells were isolated from liquid culture and extracted with each 500 µl methanol per 5 ml culture sample. Then 50 µl cell methanol extract (CME) was concentrated to 5-10 µl and spotted on the surface of a LB plate containing an indicator strain.

### Secondary metabolite production in liquid culture

For liquid cultures, each 2 x 10⁸ spores from -20 °C spore stocks in 20% glycerol of *S. coelicolor* were incubated in 50 or 60 ml DNBroth supplemented with 325 mM (final conc.) Glu in a 250 ml Erlenmeyer flask (unbaffled, with spring) shaking at 220 rpm at 30 °C overnight.

At sampling timepoints, 200 µl of a 1.2 ml culture sample (cells and culture supernatant (c + s/n)) were transferred to a 96 wells plate. The cells of the remaining 1 ml culture sample were collected by centrifugation (1 min at 13.000 rpm at room temperature) 200 µl of the (yellow pigmented) culture supernatant (s/n) were transferred to a 96 wells plate, the rest of the liquid was discarded. The cell pellet was resuspended in 1 ml colourless Tris/EDTA buffer (Kieser *et al.,* 2000) to determine the growth of the culture by photospectoscopy, if necessary, after further dilution with Tris/EDTA buffer to OD₄₅₀ < 1 (data not shown).

### Preparation of cell methanol extracts

At the same timepoints, cells of a 5 ml culture sample were collected by centrifugation (7 min, 4300 rpm, 4 °C) to prepare a cell methanol extract (CME). The liquid was removed completely, after which the cell pellet was resuspended in 500 µl methanol. After incubation on ice for 10 minutes with slight mixing, cells were spun down as described above. The obtained supernatant was collected as CME and stored at 4 °C in darkness until the last sampling timepoint (6 and 24 hours in Fig. 2 and 3, respectively). For documentation purposes, 200 µl of the CME were then transferred to a 96 wells plate.

The 96 wells plate containing the s/n, the c + s/n and the CME sample was scanned from the bottom. For reference, blank culture medium and a pure methanol sample were used. In addition to visual inspection, OD₄₅₀ of the culture supernatant and the CME samples was determined using a plate reading photospectrometer to quantify the amount of yCPK (data not shown).

### Antibacterial activity assay

To test the antibiotic activity of the methanolic cell extract, each a 50 µl aliquot of CME stored at 4 °C in darkness until the last sampling timepoint were concentrated to 5-10 µl in a SpeedVac in approximately 10 minutes. The aliquots were then dropped directly onto the surface of freshly prepared 20 ml LB plates that contained 0.4% *B. subtilis* from a 3 ml liquid LB overnight culture. The inoculated plates were allowed to dry and were incubated overnight at 30 °C. Pictures were taken from the bottom to determine inhibition of bacterial growth (Fig. 3). For Fig. 2 the CME was applied to holes in the indicator plate and the picture was taken from top.

### Results and conclusions

Results for S. *coelicolor* strain LW3 are shown in Fig. 3. LW3 was incubated in Glu-DNB and growth and secondary metabolite production were followed. CMEs were prepared from 5 ml culture samples (Fig. 3A) and tested for antibacterial activity against *B. subtilis* as described above (Fig. 3B). Bioactivity was observed from 22 hours onwards. Lack of activity at 20 h of growth, however, may be due to the low amount of extracted cells at this early time point and not due to missing abCPK production. Also, we cannot exclude that the increasing size of the inhibition zones at later time points is rather due to the increasing cell mass over time than to an increase in production of the antibiotic agent.

Cell methanol extracts also contain Red (Fig. 2 and 3; also Kieser *et al.,* 2000) and possibly other *S. coelicolor* secondary metabolites that stay inside intact cells. It is known that prodigiosins (Red) possess considerable antibiotic activity towards Gram-positive organisms (Gerber, 1975). Therefore it was important to demonstrate that the antibacterial activity observed in CMEs from liquid cultures is related to the *cpk* cluster, like the activity observed in solid cultures, and that this activity is independent from the other known *S. coelicolor* antibiotics (Act, Red, CDA).

*S. coelicolor* strains M145 (parent), LW3 (M145 *scbR2*::aac(3)IV), M1146 (M145 *Δact Δred Δcpk Δcda*) and M1147 (M145 *Δact Δred cdaPS*::aac(3)IV) were grown in liquid glutamate supplemented DNB medium, and CMEs were prepared after 22, 24, 26 and 28 hours of growth (Fig. 2A). Antibacterial activity of the CMEs was demonstrated for strains M145, LW3 and the *act red cda* mutant strain M1147, but not for the *act red cpk cda* mutant strain M1146 (Fig. 2B). These observations are consistent with the bioactivity tests performed in solid cultures (Fig. 1) and suggest that the antibiotic activity observed in cell methanol extracts from liquid cultures of *S. coelicolor* is also related to the *cpk* cluster.

### EXAMPLE 3: The antibiotic CPK compound is stable in a methanolic extract

A cell methanol extract of *S. coelicolor* strain LW3 that had been grown in Glu-DNB was prepared to test the stability of the extracted abCPK. 50 µl aliquots of the fresh CME were stored at 4 °C and room temperature (RT) or were dried-up completely in a SpeedVac, stored at -80 °C or RT, and resuspended in the same volume of methanol. Bioactivity was tested as described above, only the CMEs were applied on 0.5 mm filter disks on the indicator plate. As can be observed in Figure 4, bioactivity against *B. subtilis* was tested and could be demonstrated under all temperature conditions. Thus, the antibiotic CPK compound is stable in a methanol extract at different temperatures.

### EXAMPLE 4: The antibiotic CPK compound is smaller than 10 kDa

A sample was taken from a 28 hours liquid Glu-DNB culture supernatant (s/n) and a cell methanol extract (CME) obtained from a 45 ml culture. Both samples had a volume of 2 ml. To determine the molecular size of yCPK and abCPK, each sample was run over a 10 kDa size fractionation column (Microsep™ 10k Omega, PALL Life Sciences, Port Washington, New York, USA; centrifugation at 4 °C and ~25000 rcf).

The pigment yCPK was detected in the s/n and the column flow-through (FT) by OD₄₅₀ determination against a blank culture medium reference (Figure 5A). The fresh CME and the FT where tested for the presence of abCPK by means of bioactivity testing on plated *B. subtilis* cultures, as described in Example 2. The size fractionation membrane of the used columns was tested in the same manner, by cutting it from the columns and applying it to the *B. subtilis* plates. A blank methanol sample was used as reference.

As can be observed in Figure 5B, both the CME and the FT fraction were bioactive. No antibacterial activity of the membrane was observed. These results suggest that the CPK cluster products yCPK and abCPK are smaller than 10 kDa.

### REFERENCES

Baltz, R.H. (1998) Journal of Industrial Microbiology & Biotechnology 20: 360-363.
Baltz, R.H. (2001) Antonie van Leeuwenhoek 79: 251-259.
Bentley, S.D. (2002) Nature 417: 141-147.
Bentley, S.D. (2004) Mol Microbiol 51: 1615-1628.
Berdy, J. (2005) J Antibiot (Tokyo) 58: 1-26.
Boyle-Vavra, S. (2007) Laboratory Investigation 87: 3-9.
Butler, M.J. (2003) Applied Microbiological Biotechnology 61: 512-516. Bystrykh, L.V. (1996) J Bacteriol 178: 2238-2244.
Challis, G.L. (2003) Proc Natl Acad Sci U S A 100 Suppl 2: 14555-14561. Challis, G.L. (2008) J Med Chem 51: 2618-2628.
Feitelson, J.S. (1985) J Gen Microbiol 131: 2431-2441.
Gerber,N.N. (1975) CRC Crit Rev Microbiol 3: 469-485.
Gust, B. (2003) Proc Natl Acad Sci USA 100:1541-1546.
Hopwood, D.A. (2007) New York: Oxford University Press.
Hopwood, D.A. (1983) J Gen Microbiol 129: 3575-3579.
Hopwood, D.A. (1986) VCH, Weinheim, Germany, p. 23-33.
Inaoka T. (2004) The Journal of Biological Chemistry 279(5): 3885-3892. Kawamura, F. (1984) J Bacteriol 160: 442-444.
Kieser, T. (2000) Practical Streptomyces Genetics. Norwich, United Kingdom: John Innes Foundation, pp. 43-61.
Kim E.S. (2001) Journal of Bacteriology 183(7): 2198-2203.
Lai, C. (2002) Microbiology 148: 3365-3373.
Lakey, J.H. (1983) J Gen Microbiol 129: 3565-3573.
McEvoy, G.K. (ed.) (2009) AFHS Drug Information. American Society of Health-System Pharmacists, Inc. Bethesda, MD, USA.
Paterson, D.L. (2006) The American Journal of Medicine 119 (6 suppl. 1): S20-S28, discussion S62-S70.
Pawlik, K. (2007) Arch Microbiol 187: 87-99.
Powers, J.H. (2004) Clinical Microbiological Infections 10 suppl. 4: 23-31. Rigali, S. (2008) EMBO Rep 9: 670-675.
Romero, N.M. (1992) Nucleic Acids Research 20(11): 2767-2772.
Rudd, B.A. (1979) J Gen Microbiol 114: 35-43.
Sambrook, J. (ed.) (2001) Molecular cloning: a laboratory manual. Cold Spring Harbor Laboratory Press, New York, USA.
Slama, T.G. (2008) Critical Care 12 (suppl. 4).
Spellberg, B. (2004) Clinical Infectious Disease 38: 1279-1286.
Sweetman, S.C. (ed.) (2009) Martindale: The Complete Drug Reference. Thirty-sixth Edition. Pharmaceutical Press, London, GB.
Takano, E. (2001) Mol Microbiol 41: 1015-1028.
Tang, J.S. (2003) Int J Syst Evol Microbiol 53: 995-997.
Wang G. (2008) Applied and Environmental Microbiology 74(9): 2834-2840. Watve, M.G. (2001) Arch Microbiol 176: 386-390.
Widdick, D.A. (2006) Proc Natl Acad Sci USA 103(47):17927-32
Yonaha, K. (1992) J Biol Chem 267: 12506-12510.

## Claims

1. A method for providing an antibiotic extract, comprising:
a) providing *Streptomyces coelicolor,* or a mutant strain thereof wherein the *scF* and/or *scbR2* gene(s) is/are non-functional,
b) culturing said *Streptomyces coelicolor* under conditions allowing for production of an antibiotic compound,
c) isolating at least one cultured cell, and
d) preparing an extract from said isolated cell.

2. The method according to claim 1, comprising culturing a *Streptomyces coelicolor* mutant that contains a non-functional, preferably a deleted or disrupted, *scF* gene.

3. The method according to claim 1 or 2, wherein said mutant has functional *cpkA, cpkB, cpkC, cpkH , cpkJ, accA1, cpkF, cpkN, cpkK, scoT, scbA, scbB, scbC,* and *cpkO* genes.

4. The method according to any one of claims 1 to 3, wherein said culturing is performed in the presence of glutamate or a salt thereof, preferably wherein said glutamate or salt thereof is present in a concentration of at least 50 mM, preferably at least 100 mM, more preferably at least 300 mM.

5. The method according to any one of claims 1 to 4, wherein step d) comprises extracting isolated cultured cells with an alcohol, dimethyl sulfoxide, dimethyl formamide, dimethyl acetamide and/or 1,3-dimethyl-3,4,5,6-tetrahydro-2(1H)-pyrimidinone, preferably methanol.

6. An antibiotic extract obtainable by the method according to any one of claims 1 to 5, **characterized in that** it is biologically active against Gram-positive and Gram-negative bacteria.

7. A method for providing an antibiotic compound, comprising:
i) providing an antibiotic extract according to claim 6, and
ii) isolating at least one antibiotic compound from said extract.

8. An antibiotic compound obtainable by the method according to claim 7, **characterized in that** it is biologically active against Gram-positive and Gram-negative bacteria.

9. The extract according to claim 6 or the compound according to claim 8 for use as a medicament, preferably for the treatment of a bacterial infection, or as a selective agent.

10. Use of the extract according to claim 6 or a compound according to claim 8 in the preparation of a medicament for the treatment of a bacterial infection.

11. *A Streptomyces coelicolor* mutant, **characterized in that** it contains a non-functional *scF* gene.

12. The mutant according to claim 11, furthermore comprising a non-functional *scbR2* gene.

13. The mutant of claim 11 or 12, overexpressing the *cpkF* and/or *cpkH* genes.

14. The mutant strain according to any one of claims 11 to 13, containing functional *cpkA, cpkB, cpkC, cpkH, cpkJ, accA1, cpkF, cpkN, cpkK, scoT, scbA, scbB, scbC* and *cpkO* genes.

15. Use of a mutant strain according to claim 14 in the preparation of an antibiotic composition.
